# FASCICULE DE BREVET EUROPEEN

(11) **EP 3 579 785 B1**
(45) Date de publication et mention de la délivrance du brevet: **29.05.2024**
(21) Numéro de dépôt: 18701320.6
(22) Date de dépôt: 22.01.2018
(51) Int. Cl.: A61C 3/02, A61B 17/16, A61C 1/05

(54) **OUTIL COUPANT DU TYPE FORET CHIRURGICAL**
SCHNEIDENDES WERKZEUG VOM TYP CHIRURGISCHER BOHRER
SURGICAL DRILL-TYPE CUTTING TOOL

(30) Priorité: 07.02.2017 FR 1770120
(43) Date de publication de la demande: 18.12.2019
(73) Titulaire: Implants Diffusion International, 93100 Montreuil (FR)
(72) Inventeur: BOUKHRIS, Gilles, 75020 Paris (FR); SEBILLET, Gildas, 92320 Chatillon (FR); BOUKHRIS, Gérard, 75020 Paris (FR)
(74) Mandataire: Hirsch & Associés
(86) Numéro de dépôt international: PCT/EP2018/051466
(87) Numéro de publication internationale: WO 2018/145888

(56) Documents cités:
- EP-A1- 1 974 680
- FR-A1- 2 762 776
- FR-A1- 3 002 842
- US-A- 5 078 605
- US-A1- 2009 004 625

## Description

### Domaine de l'invention

La présente invention concerne un outil coupant tournant, de type forêt chirurgical, destiné à être porté par un contre-angle.

### Art antérieur

Parmi les outils utilisés en chirurgie, il existe des outils coupants tournants, usuellement appelés « forêts chirurgicaux », qui sont destinés à être portés par un contre-angle capable de les entraîner en rotation. De tels forêts chirurgicaux sont couramment utilisés en chirurgie dentaire ou endodontique, ou en chirurgie osseuse, par exemple pour effectuer un prélèvement d'os à des fins de greffe osseuse. Ils comprennent classiquement une queue, qui est destinée à être maintenue par le contre-angle, et une partie active, située dans le prolongement de la queue, qui est destinée à être introduite dans l'os ou la dent du patient.

Certains modèles de contre-angle utilisés pour porter les forêts chirurgicaux comprennent des systèmes d'irrigation, permettant d'amener sur le forêt chirurgical un liquide d'irrigation ou de refroidissement destiné au refroidissement du forêt chirurgical, à la lubrification et/ou à l'évacuation des copeaux du trou effectué par le forêt chirurgical.

Il est connu, par exemple du document EP 0 991 360 ou du document US 4 710 075, d'équiper des forêts chirurgicaux d'une butée de diamètre supérieur au diamètre de la partie active du forêt chirurgical. Cette butée sépare alors la partie active de la queue du forêt chirurgical, et permet ainsi de limiter l'enfoncement du forêt chirurgical à la profondeur souhaitée. Cette butée peut également servir de support d'information, par exemple par sa couleur ou par des inscriptions permettant l'identification facile du forêt chirurgical.

US2009/0004625 décrit une forêt chirurgicale comportant une queue et une partie active située dans le prolongement de la queue, la partie active présentant une goujure; et une bague entourant une portion de la partie active, une surface interne de la bague étant en contact avec la partie active, la portion de la partie active qui jouxte la queue étant entourée par la bague, la bague entourant les contours extérieurs du forêt.

### inconvénients de l'art antérieur

Il est cependant apparu que, quand un forêt chirurgical équipé d'une telle butée est utilisé avec un contre-angle permettant l'approvisionnement de liquide d'irrigation, la butée constitue un obstacle gênant l'écoulement de ce liquide d'irrigation le long du forêt chirurgical, et empêchant donc la bonne irrigation de la partie active du forêt chirurgical.

### Objectifs de l'invention

La présente invention a pour objectif de pallier ces inconvénients de l'art antérieur.

Plus précisément, la présente invention a pour objectif de fournir un forêt chirurgical, présentant une butée apte à limiter son enfoncement, et dont la partie active peut-être irriguée, de façon satisfaisante, par un liquide d'irrigation.

Un objectif particulier de l'invention est de fournir un tel forêt chirurgical capable de guider le liquide d'irrigation de telle sorte qu'il procure une irrigation utile sur la plus grande partie de la longueur de la portion active du forêt chirurgical.

Un autre objectif de l'invention, dans certains de ses modes de réalisation, est de fournir un tel forêt chirurgical qui puisse être reconnu facilement par un praticien.

### Exposé de l'invention

Ces objectifs, ainsi que d'autres qui apparaîtront plus clairement par la suite, sont atteints à l'aide d'un outil coupant du type forêt chirurgical, comprenant une queue et une partie active s'étendant dans le prolongement de ladite queue, au moins une goujure s'étendant le long de ladite partie active, qui se caractérise par le fait qu'il comprend une bague entourant une portion de ladite partie active, une surface interne de ladite bague étant en contact avec ladite partie active sans obturer ladite ou lesdites goujures.

Ainsi, les goujures n'étant pas obturées par la bague, les liquides tels que le liquide d'irrigation peuvent circuler le long de la partie active, en passant par les goujures à l'intérieur de la bague. La bague peut donc remplir les mêmes fonctions que les butées de l'art antérieur, sans empêcher la circulation correcte du liquide d'irrigation.

Avantageusement, ladite surface interne de ladite bague présente une forme de révolution.

Cette surface interne peut ainsi être en contact avec les listels de la partie active, sans s'introduire dans les goujures.

De préférence, ladite partie active présente au moins deux goujures s'étendant le long d'au moins une portion de la partie active, lesdites goujures étant séparées par des lèvres.

Avantageusement, ladite surface interne présente une gorge périphérique de diamètre supérieur au diamètre desdites lèvres, ouverte sur la face de ladite bague qui est tournée vers ladite queue.

Cette gorge permet de concentrer le liquide d'irrigation circulant sur le forêt chirurgical vers les goujures, proches du centre du forêt chirurgical.

Avantageusement, ladite gorge périphérique présente un profil concave, et la surface de ladite bague qui jouxte ladite gorge, du côté de ladite gorge qui est ouvert, présente une forme tronconique, de telle sorte que ladite surface tronconique et la surface de ladite gorge présentent, ensemble, un profil en forme d'aile de mouette.

Cette forme permet d'entraîner et de guider le liquide d'irrigation vers les goujures.

De préférence, les lèvres d'une portion de la partie active entourée par la bague présentent localement un renflement périphérique, et la surface interne de la bague présente une forme adaptée pour prendre appui sur ce renflement.

Ce renflement permet de bloquer efficacement la translation de la bague le long de la partie active du forêt chirurgical.

Avantageusement, ladite ou lesdites goujures présentent, sur une portion de ladite partie active entourée par ladite bague, une orientation hélicoïdale dans un sens permettant d'entraîner vers la partie active un fluide présent autour de ladite queue, quand ledit outil coupant est entraîné en rotation dans son sens normal de rotation.

Ainsi, les goujures tendent à entraîner le liquide vers la pointe du forêt chirurgical, avec une vitesse d'autant plus importante que la vitesse de rotation du forêt chirurgical est élevée.

Selon un autre mode de réalisation, ladite bague comprend au moins une portion formant une pale ou une hélice, orientée dans un sens permettant d'entraîner vers la partie active un fluide présent autour de ladite queue, quand ledit outil coupant est entraîné dans son sens normal de rotation.

La bague elle-même peut dans ce cas contribuer à entraîner le liquide d'irrigation vers la pointe du forêt.

Selon un mode de réalisation, ladite bague est montée de façon amovible sur ladite partie active.

De façon avantageuse, ladite partie active est configurée pour recevoir ladite bague en plusieurs positions, ou pour recevoir plusieurs bagues.

Selon un autre mode de réalisation, ladite bague est montée de façon fixe sur ladite partie active.

Avantageusement, ladite bague est constituée de matériau métallique.

De façon avantageuse, ladite bague porte des signes visibles, aptes à donner une information au praticien.

### Liste des figures

L'invention sera mieux comprise à la lecture de la description suivante de modes de réalisation préférentiels, donnée à titre de simple exemple figuratif et non limitatif, et accompagnée de la figure suivante :

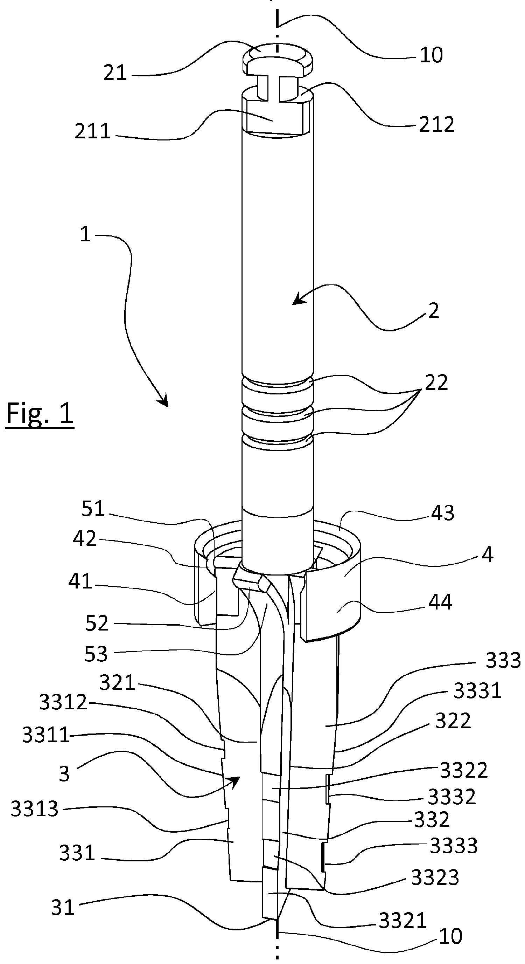

- la figure 1 est une vue en perspective d'un forêt chirurgical selon un mode de réalisation préférentiel de l'invention, comprenant une bague qui est représentée en écorché.

### Description détaillée de modes de réalisation de l'invention

La figure 1 représente un outil coupant du type forêt chirurgical. Ce forêt chirurgical 1 comprend classiquement une queue 2, également appelée « queue dentaire », et une partie active 3, située dans le prolongement de la queue 2.

### La queue

La queue 2 est destinée à être insérée et maintenue dans un contre-angle, ou dans tout autre instrument permettant de maintenir le forêt chirurgical 1 et de l'entraîner en rotation. De façon classique, cette queue 2 présente une forme générale cylindrique, centrée sur un axe 10 appelé par la suite « axe du forêt ». La queue 2 présente, à proximité de sa première extrémité 21 formant une extrémité du forêt chirurgical 1, un méplat 211 et une rainure périphérique 212 permettant son maintien et son entraînement en rotation par le contre-angle.

Dans certains modes de réalisation possibles, cette queue 2 peut présenter des informations destinées au praticien, qui peut être sous la forme d'un code couleur, par exemple sous la forme de marquages circulaires, de gravures telles que des stries périphériques, ou de signes indiquant une référence ou une information sur le forêt. Ainsi, la queue 2 représentée sur la figure 1 présente trois stries périphériques 22, lui permettant d'être reconnue aisément par le praticien.

### La partie active

Cette queue 2 est prolongée, au-delà de sa seconde extrémité opposée à sa première extrémité 21, par la partie active 3 du forêt chirurgical qui s'étend jusqu'à la pointe 31 du forêt chirurgical. Cette partie active 3 est destinée à être introduite dans la dent ou l'os d'un patient, pour y effectuer un usinage.

Cette partie active 3 présente une section en croix, présentant quatre lèvres séparant quatre goujures. Trois de ces lèvres, référencées 331, 332 et 333, sont visibles sur la figure 1. Chaque goujure est définie entre deux lèvres voisines. Deux de ces goujures sont visibles sur la figure 1 : la goujure 321 qui s'étend entre la lèvre 331 et la lèvre 332, et la goujure 322 qui s'étend entre la lèvre 332 et la lèvre 333. Dans le mode de réalisation représenté, les quatre lèvres s'étendent de façon sensiblement rectiligne, selon deux plans perpendiculaires sécants le long de l'axe du forêt 10.

Il est cependant possible, dans d'autres modes de réalisation de l'invention, que les goujures présentent une orientation hélicoïdale, dont l'hélice présente par exemple une pente comprise entre 0° et 150°, dans un sens ou dans l'autre. De même, il est possible que la partie active 3 comprenne un nombre différent de goujures, une seule goujure étant nécessaire pour la mise en oeuvre de la solution proposée.

La surface externe de chaque lèvre, respectivement 331, 332 et 333, forme le listel de la lèvre, respectivement 3311, 3321 et 3331. Sur la plus grande portion de la partie active 3, ces listels s'inscrivent dans une enveloppe formant un tronc de cône, centré sur l'axe du forêt 10. Dans d'autre modes de réalisation possibles, cette enveloppe peut être de forme cylindrique, cylindro-cônique, cônique, étagée, ou de toute autre forme connue de l'homme du métier.

Dans le mode de réalisation représenté, des dents 3312, 3313, 3322, 3323, 3332 et 3333 sont usinées dans la surface des listels 3311, 3321 et 3331. Dans d'autres modes de réalisation, les listels peuvent présenter des configurations différentes. Ils peuvent par exemple présenter des gorges, hélicoïdales ou dans des plans perpendiculaires à l'axe du forêt 10.

De façon générale, la partie active du forêt chirurgical peut présenter toute forme connue de l'homme du métier pour de tels forêts chirurgicaux.

### La bague

A son extrémité opposée à la pointe 31 du forêt chirurgical, la portion de la partie active 3 qui jouxte la queue 2 est entourée par une bague 4. Il est à noter que cette bague 4 est représentée en écorché sur la figure 1, afin de montrer sa forme interne et la forme de la portion de la partie active 3 qu'elle entoure. La bague 4 représentée sur la figure 1 présente ainsi, en réalité, une forme de révolution centrée sur l'axe du forêt 10. La bague 4 entoure les contours extérieurs du forêt chirurgical, formés par le listel des lèvres de la partie active, sans obturer les goujures.

Ainsi, la présence de cette bague 4 n'empêche pas l'écoulement de liquide le long du forêt chirurgical 1. Un liquide s'écoulant le long de la queue 2 dans la direction de la pointe 31, par exemple sous l'effet de la gravité, peut ainsi s'écouler facilement le long de la partie active 3, en s'introduisant dans les goujures pour passer dans la bague 4.

La bague 4, qui présente de préférence un diamètre supérieur au diamètre maximal de la partie active 3, peut servir de butée limitant l'enfoncement de la partie active, et sécurisant donc l'usage du forêt chirurgical 1. Le forêt chirurgical 1 comprenant la bague 4 présente ainsi les avantages des forêts de l'art antérieur pourvus de butées tout en permettant l'irrigation satisfaisante de la partie active par un liquide d'irrigation.

### Le haut de la partie active

La portion de la partie active 3 qui est entourée par la bague 4 présente avantageusement une configuration particulière. Ainsi, sur la portion de la partie active 3 qui jouxte la queue 2, le listel des lèvres présente un renflement périphérique, conférant localement à l'enveloppe dans laquelle s'inscrit la partie active 3 un diamètre plus important. Sur la figure 1, on peut ainsi voir les renflements 51 et 52, respectivement sur les portions des lèvres 331 et 332 qui jouxtent la queue 2.

Ce renflement forme, avantageusement, un appui pour la bague 4, qui présente une forme interne complémentaire. Ainsi, il empêche la bague 4 de se déplacer par glissement le long du forêt chirurgical 1, vers l'extrémité 21. Dans d'autres modes de réalisation, ce renflement peut ne pas apparaître. Ainsi, la partie active peut présenter d'autres formes, telles que des rainures, permettant un blocage de la translation de la bague 4. Dans d'autres modes de réalisation encore, la bague 4 peut être fixée sur la partie active 3 par d'autres moyens, par exemple par collage.

Par ailleurs, les goujures et les lèvres qui les séparent présentent localement, au niveau de la portion de la partie active 3 qui est entourée par la bague 4, une forme hélicoïdale. Cette forme hélicoïdale est particulièrement visible, sur la figure 1, par l'inclinaison de la portion 53 de la lèvre 322. L'hélice ainsi formée présente avantageusement une orientation permettant, quand le forêt chirurgical 1 est entraîné en rotation dans son sens normal de rotation, d'entraîner et d'aspirer le liquide d'irrigation se trouvant autour de la queue 2 pour le propulser dans les goujures de la partie active 3. Ainsi, le liquide d'irrigation est entraîné vers la pointe 31, avec une vitesse augmentant avec la vitesse de rotation du forêt chirurgical 1.

Dans d'autres modes de réalisation possibles de l'invention, les goujures peuvent ne pas présenter cette forme d'hélice. Il est notamment possible, dans un mode de réalisation particulier, que la bague 4 elle-même présente des portions formant des pales ou une hélice, permettant d'obtenir le même effet de propulsion du liquide d'irrigation se trouvant autour de la queue 2 vers les goujures de la partie active 3. Il est également possible, dans encore d'autres modes de réalisation, que le liquide d'irrigation ne s'écoule dans les goujures que par une propulsion conférée par le système d'irrigation du contre-angle, ou encore sous l'effet de la gravité.

### Face interne de la bague

La face interne de la bague 4, qui est en contact avec la partie active 3 du forêt chirurgical, présente de préférence une forme de révolution. Dans le mode de réalisation représenté, elle présente, sur la plus grande partie de sa longueur, une forme cylindrique 41 pour s'adapter à la surface des listels, qui s'inscrit dans une forme cylindrique sur cette portion de la partie active 3.

Au niveau des renflements 51 et 52 de la partie active, la face interne de la bague 4 présente une gorge concave 42 lui permettant de prendre appui sur les renflements 51 et 52. Cette gorge est avantageusement de taille plus importante que les renflements, et est ouverte dans la direction de la queue 2. Elle se prolonge par une portion inclinée 43, de forme tronconique, qui forme la face de la bague 4 orientée vers la queue 2. La gorge concave 42 et la portion inclinée 43 forment ensemble un profil dit « en aile de mouette » connu notamment pour les formes des ailes d'avions ou des carènes de bateaux. Ce profil ouvert vers la queue 2 permet de concentrer vers les goujures de la partie active 3 le liquide d'irrigation s'écoulant le long de la queue 2 du forêt chirurgical 1.

Dans d'autres modes de réalisation possibles, la face interne de la bague 4 peut présenter d'autres formes. Elle peut par exemple présenter une ou plusieurs gorges concaves ou convexes.

### Forme externe de la bague

La bague 4 peut être fabriquée en métal ou en tout autre matériau approprié. Son épaisseur, dans la direction définie par l'axe de forêt 10, est avantageusement supérieure à 1 millimètre, et peut être de plusieurs millimètres. Son diamètre est avantageusement supérieur à 2 millimètres.

Cette bague 4 peut être de la couleur naturelle du matériau qui la forme, ou être colorée de toute autre couleur permettant de constituer un signe de reconnaissance destiné au praticien. Sa face externe 44, dans le mode de réalisation représenté, est cylindrique. Cette face externe peut avantageusement recevoir tout signe de reconnaissance, tel que des stries horizontales ou verticales, des signes ou des couleurs, permettant d'informer le praticien sur le numéro de série, de lot, la longueur du forêt chirurgical, sa référence, sa longueur active ou son diamètre, ou plus généralement pour donner toute information utile au praticien.

Il est également possible, dans un mode de réalisation particulier de l'invention, que la bague 4 présente des perçages latéraux entre sa face interne et sa face externe, pour laisser passer le liquide d'irrigation.

La bague 4 peut être assemblée sur la partie active du forêt par emmanchement ou par clippage. Cet assemblage peut être définitif, pour rendre la bague fixe, ou peut être réversible pour rendre la bague amovible. Il est possible, dans certains modes de réalisation, que le forêt chirurgical soit configuré pour recevoir une bague dans plusieurs positions sur la partie active 3, ou qu'il puisse recevoir plusieurs bagues. Dans certains modes de réalisation possibles, les renflements de la partie active 3 peuvent servir pour clipper une bague amovible ou, de façon alternative, une butée d'un type différent.

## Revendications

1. Un outil coupant du type forêt chirurgical (1) destiné à être porté par un contre-angle capable de l'entraîner en rotation comportant:
- une queue (2) et une partie active (3) située dans le prolongement de la queue (2);
- la queue (2) présentant à proximité de sa première extrémité (21) formant une extrémité du forêt chirurgical (1), un méplat (211) une rainure périphérique (212) permettant son maintien et son entraînement en rotation par le contre-angle;
- la partie active (3) présentant une section en croix, comportant quatre lèvres séparant quatre goujures (321, 322); l'outil comportant
- une bague entourant une portion de ladite partie active (3), une surface interne de ladite bague (4) étant en contact avec ladite partie active (3);
- la portion de la partie active (3) qui jouxte la queue (2) étant entourée par la bague (4), laquelle entoure les contours extérieurs du forêt, formés par le listel des lèvres de la partie active, sans obturer les goujures et ainsi sans empêcher l'écoulement de liquide, tel produit d'irrigation ou de refroidissement sur le forêt (1);
- la face interne de la bague (4) présentant une gorge concave (42) ouverte dans la direction de la queue (2) et se prolongeant par une portion inclinée (43), de forme tronconique, qui forme la face de la bague (4) orientée vers la queue, cette gorge concave (42) et la portion inclinée (43) formant ensemble un profil dit «en aile de mouette» qui permet de concentrer vers les goujures de la partie active (3) le liquide d'irrigation ou de refroidissement s'écoulant le long de la queue (2) du forêt.

2. L'outil coupant du type forêt chirurgical selon la revendication 1, dans lequel ladite gorge concave (2) est une gorge périphérique (42) de diamètre supérieur au diamètre desdites lèvres, ouverte sur la face de ladite bague (4) qui est tournée vers ladite queue (2).

3. L'outil coupant du type forêt chirurgical selon la revendication 1, dans lequel ladite surface interne de ladite bague (4) présente une forme de révolution.

4. L'outil coupant du type forêt chirurgical selon la revendication 1, dans lequel ladite partie active (3) présente au moins deux goujures (321, 322) s'étendant le long d'au moins une portion de la partie active (3), lesdites goujures (321, 322) étant séparées par des lèvres (331, 332, 333).

5. L'outil coupant du type forêt chirurgical selon la revendication 1, dans lequel les lèvres (331, 332, 333) d'une portion de la partie active entourée par la bague présentent localement un renflement périphérique (51, 52), et la surface interne de la bague (4) présente une forme adaptée pour prendre appui sur ce renflement.

6. L'outil coupant du type forêt chirurgical selon la revendication 1, dans lequel ladite ou lesdites goujures (321, 322) présentent, sur une portion de ladite partie active (3) entourée par ladite bague (4), une orientation hélicoïdale dans un sens permettant d'entraîner vers la partie active (3) un fluide présent autour de ladite queue (2), quand ledit outil coupant est entraîné en rotation dans son sens normal de rotation.

7. L'outil coupant du type forêt chirurgical selon la revendication 1, dans lequel ladite bague (4) comprend au moins une portion formant une pale ou une hélice, orientée dans un sens permettant d'entraîner vers la partie active (3) un fluide présent autour de ladite queue (2), quand ledit outil coupant est entraîné dans son sens normal de rotation.

8. L'outil coupant du type forêt chirurgical selon la revendication 1, dans lequel ladite bague (4) est montée de façon amovible sur ladite partie active (3).

9. L'outil coupant du type forêt chirurgical selon la revendication 1, dans lequel ladite partie active (3) est configurée pour recevoir ladite bague (4) en plusieurs positions ou pour recevoir plusieurs bagues.

10. L'outil coupant du type forêt chirurgical selon la revendication 1, dans lequel ladite bague (4) est montée de façon fixe sur ladite partie active (3).

11. L'outil coupant du type forêt chirurgical selon la revendication 1, dans lequel ladite bague (4) est constituée de matériau métallique.

12. L'outil coupant du type forêt chirurgical selon la revendication 1, dans lequel ladite bague porte des signes visibles, aptes à donner une information au praticien.

## Patentansprüche

1. Schneidwerkzeug des Typs chirurgischer Bohrer (1), das dazu bestimmt ist, von einem Winkelstück getragen zu werden, das geeignet ist, es in Rotation anzutreiben, umfassend:
- ein Endstück (2) und einen aktiven Abschnitt (3), der sich in der Verlängerung des Endstücks (2) befindet;
- wobei das Endstück (2) in der Nähe seines ersten Endes (21), das ein Ende des chirurgischen Bohrers (1) bildet, eine Abflachung (211) und eine Umfangsnut (212) aufweist, die seinen Halt und seinen Antrieb in Rotation durch das Winkelstück ermöglicht;
- wobei der aktive Abschnitt (3) einen kreuzförmigen Querschnitt aufweist, der vier Lippen umfasst, die vier Spannuten (321, 322) trennen,
wobei das Werkzeug umfasst:
- einen Ring, der einen Teil des aktiven Abschnitts (3) umgibt, wobei eine Innenfläche des Rings (4) mit dem aktiven Abschnitt (3) in Kontakt ist;
- wobei der Teil des aktiven Abschnitts (3), der an das Endstück (2) angrenzt, von dem Ring (4) umgeben ist, welcher die äußeren Konturen des Bohrers, die von dem Steg der Lippen des aktiven Abschnitts gebildet sind, umgibt, ohne die Spannuten zu verlegen, und somit ohne das Abfließen von Flüssigkeit, wie ein Produkt zur Bewässerung oder Kühlung auf dem Bohrer (1), zu behindern;
- wobei die Innenfläche des Rings (4) eine konkave Nut (42) aufweist, die in Richtung des Endstücks (2) offen ist und sich durch einen geneigten Abschnitt (43) von kegelstumpfartiger Form verlängert, der die zu dem Endstück gerichtete Fläche des Rings (4) bildet, wobei diese konkave Nut (42) und der geneigte Abschnitt (43) gemeinsam ein so genanntes "Möwenflügel"-Profil bilden, das es ermöglicht, die Bewässerungs- oder Kühlflüssigkeit, die entlang des Endstücks (2) des Bohrers abfließt, zu den Spannuten des aktiven Abschnitts (3) hin zu konzentrieren.

2. Schneidwerkzeug des Typs chirurgischer Bohrer nach Anspruch 1, bei dem die konkave Nut (2) eine Umfangsnut (42) mit einem größeren Durchmesser als der Durchmesser der Lippen ist, die auf der Seite des Rings (4), die zu dem Endstück (2) gerichtet ist, offen ist.

3. Schneidwerkzeug des Typs chirurgischer Bohrer nach Anspruch 1, bei dem die Innenfläche des Rings (4) eine Umdrehungsform aufweist.

4. Schneidwerkzeug des Typs chirurgischer Bohrer nach Anspruch 1, bei dem der aktive Abschnitt (3) mindestens zwei Spannnuten (321, 322) aufweist, die sich entlang mindestens eines Teils des aktiven Abschnitts (3) erstrecken, wobei die Spannuten (321, 322) durch Lippen (331, 332, 333) getrennt sind.

5. Schneidwerkzeug des Typs chirurgischer Bohrer nach Anspruch 1, bei dem die Lippen (331, 332, 333) eines Teils des aktiven Abschnitts, der von dem Ring umgeben ist, lokal einen Umfangswulst (51, 52) aufweisen, und die Innenfläche des Rings (4) eine Form aufweist, die dazu geeignet ist, auf diesem Wulst zur Auflage zu gelangen.

6. Schneidwerkzeug des Typs chirurgischer Bohrer nach Anspruch 1, bei dem die Spannut(en) (321, 322) auf einem Teil des aktiven Abschnitts (3), der von dem Ring (4) umgeben ist, eine spiralförmige Ausrichtung in eine Richtung aufweisen, die es ermöglicht, ein Fluid, das um das Endstück (2) vorhanden ist, zu dem aktiven Abschnitt (3) zu treiben, wenn das Schneidwerkzeug in Rotation in seiner normalen Rotationsrichtung angetrieben wird.

7. Schneidwerkzeug des Typs chirurgischer Bohrer nach Anspruch 1, bei dem der Ring (4) mindestens einen Teil aufweist, der ein Schaufelblatt oder eine Schraube bildet, die in eine Richtung ausgerichtet ist, die es ermöglicht, ein Fluid, das um das Endstück (2) vorhanden ist, zu dem aktiven Abschnitt (3) zu treiben, wenn das Schneidwerkzeug in seiner normalen Rotationsrichtung angetrieben wird.

8. Schneidwerkzeug des Typs chirurgischer Bohrer nach Anspruch 1, bei dem der Ring (4) abnehmbar auf dem aktiven Abschnitt (3) montiert ist.

9. Schneidwerkzeug des Typs chirurgischer Bohrer nach Anspruch 1, bei dem der aktive Abschnitt (3) eingerichtet ist, um den Ring (4) in mehreren Positionen aufzunehmen oder um mehrere Ringe aufzunehmen.

10. Schneidwerkzeug des Typs chirurgischer Bohrer nach Anspruch 1, bei dem der Ring (4) fest auf dem aktiven Teil (3) montiert ist.

11. Schneidwerkzeug des Typs chirurgischer Bohrer nach Anspruch 1, bei dem der Ring (4) aus einem metallischen Material hergestellt ist.

12. Schneidwerkzeug des Typs chirurgischer Bohrer nach Anspruch 1, bei dem der Ring sichtbare Zeichen trägt, die geeignet sind, dem Arzt eine Information zu liefern.

## Claims

1. A surgical drill type cutting tool (1) intended to be carried by a contra-angle handpiece capable of rotating it comprising:
- a shaft (2) and an active part (3) located in the extension of the shaft
- the shaft (2) having near its first end (21) forming one end of the surgical drill (1), a flat (211) and a peripheral groove (212) allowing it to be held and rotated by the contra-angle ;
- the active part (3) having a cross-shaped section, comprising four lips separating four flutes (321, 322); the tool comprising
- a ring surrounding a portion of said active part (3), an internal surface of said ring (4) being in contact with said active part (3);
- the portion of the active part (3) which adjoins the shaft (2) being surrounded by the ring (4), which surrounds the external contours of the drill, formed by the listel of the lips of the active part, without blocking the flutes and thus without preventing the flow of liquid, such as irrigation or cooling product over the drill (1);
- the internal face of the ring (4) having a concave groove (42) open in the direction of the shaft (2) and being extended by an inclined portion (43), of frustoconical shape, which forms the face of the ring (4) oriented towards the shaft, this concave groove (42) and the inclined portion (43) together forming a so-called "gull wing" profile which allows the irrigation or cooling liquid flowing along the shaft (2) of the drill to be concentrated towards the flutes of the active part (3).

2. The surgical drill type cutting tool according to claim 1, wherein said concave groove (2) is a peripheral groove (42) of diameter greater than the diameter of said lips, open on the face of said ring (4) which is facing said shaft (2).

3. The surgical drill type cutting tool according to claim 1, wherein said internal surface of said ring (4) has a shape of a body of revolution.

4. The surgical drill type cutting tool according to claim 1 wherein said active part (3) has at least two flutes (321, 3 22) extending along at least one portion of the active part (3), said flutes (321, 322) being separated by lips (331, 332, 333).

5. The surgical drill type cutting tool according to claim 1, in which the lips (331, 332, 333) of a portion of the active part surrounded by the ring locally have a peripheral bulge (51, 52), and the internal surface of the ring (4) has a shape adapted to rest on this bulge.

6. The surgical drill type cutting tool according to claim 1, in which said flute(s) (321, 322) have, on a portion of said active part (3) surrounded by said ring (4), a helical orientation in a direction making it possible to drive towards the active part (3) a fluid present around said shaft (2), when said cutting tool is rotated in its normal direction of rotation.

7. The surgical drill type cutting tool according to claim 1, in which said ring (4) comprises at least one portion forming a vane or an impeller, oriented in a direction allowing it to drive a fluid present around said shaft (2) towards the active part (3), when said cutting tool is driven in its normal direction of rotation.

8. The surgical drill type cutting tool according to claim 1, wherein said ring (4) is removably mounted on said active part

9. The surgical drill type cutting tool according to claim 1, wherein said active part (3) is configured to receive said ring (4) in several positions or to receive several rings.

10. The surgical drill type cutting tool according to claim 1, wherein said ring (4) is fixedly mounted on said active part (3).

11. The surgical drill type cutting tool according to claim 1, wherein said ring (4) is made of metallic material.

12. The surgical drill type cutting tool according to claim 1, wherein said ring bears visible signs, capable of providing information to the practitioner.
